# EUROPEAN PATENT SPECIFICATION

(11) **EP 3 743 117 B1**
(45) Date of publication and mention of the grant of the patent: **27.03.2024**
(21) Application number: 19701242.0
(22) Date of filing: 25.01.2019
(51) Int. Cl.: A61L 2/08, A61L 2/00

(54) **PHOTOBIOMODULATION DECONTAMINATION METHOD**
PHOTOBIOMODULATIONS-DEKONTAMINATIONSVERFAHREN
METHODE DE DECONTAMINATION PAR PHOTOBIOMODULATION

(30) Priority: 26.01.2018 EP 18305067
(43) Date of publication of application: 02.12.2020
(73) Proprietor: URGO RECHERCHE INNOVATION ET DEVELOPPEMENT, 21300 Chenôve (FR); Universität Heidelberg, 69117 Heidelberg (DE)
(72) Inventor: BOUSCHBACHER, Marielle, 21220 CHAMBOLLE-MUSIGNY (FR); STEINBRUNN, Julien Denis Marie, 21380 MESSIGNY-ET-VANTOUX (FR); LAVIGNE, Louis, 75020 PARIS (FR); GRETZ, Norbert, 68159 Mannheim (DE); BECKER, Anja, 68165 MANNHEIM (DE); HULLEN, Andreas, 69123 Heidelberg (DE); KLAPCZYNSKI, Anna, 68167 MANNHEIM (DE); KUCH, Natalia, 64295 DARMSTADT (DE); MU, Yifei, 69189 MANNHEIM (DE)
(74) Representative: Plasseraud IP
(86) International application number: PCT/EP2019/051923
(87) International publication number: WO 2019/145519

(56) References cited:
- WO-A1-2007/012875
- WO-A1-2017/205578
- WO-A2-2005/034855
- US-A1- 2014 343 478
- US-A1- 2017 080 117

## Description

### TECHNICAL FIELD

This invention relates to an *in-vitro* method for inhibiting growth and reducing number of contaminating and/or pathogenic agents, notably for the treatment of medium (such as the air, used waters, dairy products, drinks or beverages) or such as packing, wrapping, food products, and cleaning and/or domestic devices) preferably through a photobiomodulation mean.

### BACKGROUND OF THE INVENTION

The disclosure relates to the use of visible light, in the wavelength (λ) range of 435 to 520 nanometers (nm), in combination with highly specific fluence and power density to create a specific light, which can reduce the growth and the number of contaminating and/or pathogenic agents on any support or in any medium.

Bacteria, such as *Pseudomonas aeruginosa, Staphylococcus aureus* or *Escherischia coli,* or more generally, contaminating and/or pathogenic agents, are responsible for the development of various human and animal diseases or disturbances of their global Health and well-being. For example, the growth and the proliferation of these bacteria's species can lead to urinary, pulmonary, digestive or skin infections or disorders of the skin or wounds.

Generally, different forms of light can be used in many different applications. Through the delivery of specific wavelengths of light, effects such as the inactivation of microorganisms such as bacteria, yeasts or fungi, viruses and parasites can be accomplished.

Some specific wavelengths of visible light can be used for active reasons beyond general illumination. For example, activation of fluorescent materials can be achieved with approximately 400 to approximately 420 nm range, similar to UVA, or a "black light," curing of plastics can be achieved with approximately 380 to approximately 420 nm light, heat delivery can be achieved with near-infrared approximately 650 to approximately 700 nm light, and inactivation of bacteria can be achieved with near-infrared approximately 650 to approximately 700 nm light. Regarding inactivation of bacteria, millions of hospital patients contract a hospital-acquired infection (HAI) from bacterial, viral, or fungal microorganisms. Environmental contamination in hospital environments is a key factor in the source of these HAIs, among others. Current methods of attacking environmental contamination range widely, from traditional mopping and surface cleaning to the use of burst ultraviolet (UV) and hydrogen peroxide vapor. Yet, in full force application, infections are still a reality in almost every hospital.

In the cultivation of livestock and agricultural products, contamination from bacteria, fungi, or viruses can cause losses of animal life, plant life, and/or spoilage of rendered products. Common production practices now pack animals and plants densely for efficiency, in terms of space and finances, yet contamination from microorganisms can spread rapidly in such an environment, with infection spreading between plants or animals. Currently, there is extensive use of pesticides, antibiotics, and chemical cleaners to prevent loss of final product by preventing contamination of animals or agriculture products, yet animal and plant losses, final product losses, and the unknown distribution of contaminated final products is still an issue faced by the industry. Thus, there is a continuing need for better methods to control microorganisms in the cultivation environment and processing facilities to prevent loss of final product.

In the retail sale of food, fresh products are commonly displayed to customers in the shopping environment. In many retail stores, the products are stored on shelves and in cases with viewing windows. Many of these products are considered perishable, with a very short shelf life like meat, produce, or fish. The short shelf life of these items is due to the degradation of the quality of the product over the time displayed. This degradation of the product is caused by a variety of factors: breakdown of cells or molecules due to aging, loss of water or other volatile components into the air, or spoilage based on bacterial, fungal, or viral contamination.

Controlled environments are required for many purposes, such as the preservation of food products, the aging of goods, such as wines, liquors, and tobacco products, general prevention of contamination during many industrial processes, or in medical treatments such as during wound healing process. Such environments are protected and controlled in many different manners, including in terms of air quality, temperature, humidity, and particle count.

Perishable food is commonly stored in refrigerated enclosures to slow degradation of the food and to slow bacterial growth and proliferation that can cause food spoilage and food sickness. While refrigeration alone can extend food life and quality, compared with room temperature storage, bacteria and molds can still be common destroyers of food in these environments, in a home refrigerator just as in an industrial meat locker.

In the field of cosmetics or pharmaceuticals, products must meet preservation standards in regards of microbial proliferation to ensure sufficient shelf life and microbial cleanliness of the products. One of the methods for meet this preservation standard is to include preservatives in the product, such as parabens or phenoxyethanol.

However, these preservatives can be poorly tolerated and may be considered potentially endocrine disruptors. Therefore, the prevention of the growth and proliferation of bacteria or fungi within cosmetic or dermatologic formulations without using preservatives would be of high value. This is even more important for customer or patient with highly reactive skin or atopic dermatitis who have to use formulations without any preservatives as they can cause skin irritation or even allergy.

Humidors are humidity controlled environments, commonly associated with the storage or aging of cigars and tobacco products, that maintain moisture content at a set level for the items stored in the enclosure. However, bacterial and mold spoilage of these goods can occur in the event of contamination, resulting in the loss of what is typically a high value product.

In clean rooms, efforts are made to control the amount of particles in the air in a given enclosure. Most of them function by continuously pumping in filtered air and forcing the exodus of airborne particles. Bacterial growth and number and the generation of bacteria or mold spores from contaminated sites can continuously generate particles in the environment that can be difficult to prevent and cause costly contamination issues in high-value products undergoing processing or storage in the environment.

In a food preparation environment (e.g., restaurants, industrial kitchens, fast food, prepared goods store, for direct sale or delivery to the consumer/customer) bacteria, fungi, parasites and/or viruses pose issues of spoilage, pathogenic contamination, and infection, and can be a serious issue for the establishment. These contamination issues can come from a large variety of sources in such an open environment: e.g., personnel, customers, raw materials, air systems, and water. While many cleaning practices have typically been implemented at these sites, contamination and infection outbreaks are still seen. Typically, these contamination issues are only noticed after the damage is done, when inventory is spoiled or customers are sick.

Another interesting use of light is linked to the clothing items and the necessity to clean any cloth or shoe from any contaminating agent.

In the medical field, the prevention of growth and proliferation of contaminating and/or pathogenic agents is mostly obtained by the use of topically applied antiseptic agents.

UV light can be used for disinfection in an industrial or medical environment, but its reductive effects on any contaminating and/or pathogenic agents' growth and proliferation could still be further improved.

The effect of light emitting in the UV or violet wavelength has already been disclosed in the patent application WO 2009/056838. This document describes a device that emits in specific wavelength comprised between 380 and 420 nm, preferably at 405 nm, said device comprising LEDs having a power density of 10 mW/cm² and providing an effective fluence of at least 40 J/cm² for reducing the growth and the number of different species of bacteria. WO 2017/205578 describes a system and method for photoeradication of microorganisms from a target wherein a light source is configured to irradiate the target with purple or blue light in a pulsed mode of irradiation at an average irradiance that preferably ranges from 0.1 mW/cm2 to 20 mW/cm2.

Nevertheless, UV light is known as being a human carcinogen and may cause DNA damages such as mutations.

In addition, a publication made by Guffey and Wilborn (2006) "In Vitro Bactericidal Effects of 405-nm and 470-nm Blue Light", in Photomedicine and Laser Surgery. 24(6): 684-688; discloses the difficulty of providing a desired reduction of growth and number of different species of bacteria using different wavelengths, typically using dominant emission wavelength at 405 and 470 nm (within violet and blue spectral respectively) and using different fluence values. Indeed, this document describes how the number of colonies of different species of bacteria chosen from *S. aureus* (Gram-positive bacteria) or *P. aeruginosa* (Gram-negative bacteria) can evolve differently when exposed to different wavelength and different fluence values. More precisely, this document exhibits a drastic reduction of the number of colonies of *P. aeruginosa* or *S. aureus* at a wavelength of 405 nm, whatever the fluence. On the contrary, at a wavelength of 470 nm, the number of colonies of *P. aeruginosa* does not decrease with the increase of fluence. These effects are not similarly reproduced against *S. aureus.*

It appears therefore difficult to find a standard device that permits to significantly reduce the growth and the number of contaminating and/or pathogenic agents of a treated support or medium, that would provide reproducible results whatever the strain (ie providing a reduction of growth and number of agents for example to any Gram-positive or Gram-negative specie). There would therefore be a need for a standard device capable of providing a significant and reproducible reduction of the growth and proliferation of contaminating and/or pathogenic agents, whatever the nature or specie of said agents, said device being safe and harmless towards human health.

### SUMMARY OF THE INVENTION

It was surprisingly discovered that the growth and the number reduction of contaminating and/or pathogenic agents (including microorganisms (such as bacteria, yeasts or fungi), organisms (such as parasites, dust mite, worm, and louse) and viruses) obtained with blue light could be significantly improved by irradiating said contaminating and/or pathogenic agents with a specific wavelength range light and under specific conditions. In particular, contaminating and/or pathogenic agents is preferably a microorganism such as bacteria, yeasts or fungi, preferably bacteria. More preferably, the contaminating and/or pathogenic agent is a bacteria, in particular a Gram-positive or Gram-negative bacteria, preferably chosen from *S*. *aureus* and *P. aeruginosa.* Blue light is generally known for its anti-proliferative effect, but the inventors demonstrated that the anti-contaminating and/or anti-infectious effects could be further significantly improved using a specific dominant emission wavelength, irradiance and fluence, leading to a significant growth and number reduction of contaminating and/or pathogenic agents with applications to the treatment of liquid or fluid medium such as, the air, used water, dairy products, drinks or beverages or supports such as surface decontamination or disinfection of skin, wounds, mucosa, or such as packing, wrapping, food products, and cleaning and/or domestic devices preferably through a photobiomodulation means.

Photodynamic therapy is a method that uses a photosensitizer, or photosensitizing agent, which is disposed or injected near the treated medium or support, more specifically near the skin, mucosa or the wound and activated by a light at a specific wavelength. Photosensitizers have the ability to interact with contaminating and/or pathogenic agents when exposed to a light at a specific wavelength. Photodynamic therapy is thus an indirect phototherapy because the light is provided to the photosensitizer to treat a medium or support containing a contaminating and/or pathogenic agent, but the light is not directly provided to this medium or support.

Photobiomodulation is a method to provide a biological effect on support or in a medium, directly, which means without the need of providing any product or composition to transpose or potentialize the biological effect engendered by the light source. This method can be distinguished from the photodynamic therapy which needs absolutely and every time the intervention of an intermediate product (photosensitizer or a photosensitizing agent) between the light source and support or medium to potentialize the biological effect of the light on said support or medium. In other words, in photobiomodulation, light has a direct effect on support or on the contaminating and/or pathogenic agent whereas, in photodynamic therapy, light has an indirect effect on said support or contaminating and/or pathogenic agents via the activated photosensitizer. As mentioned in the technical field above, the present invention is preferably directed to photobiomodulation.

The unexpected technical effect is achieved with an *in-vitro* method for inhibiting growth and reducing number of contaminating and/or pathogenic agents, the method comprising the step of: exposing the contaminating and/or pathogenic agents with a light source device (10) comprising a light emitting element (12) for emitting a blue light having a wavelength ranging from 435 to 520 nm, having a power density greater than 20 mW/cm² and
the light source device (10) being configured to provide an effective fluence
to the contaminating agent and/or pathogenic agents greater than 11 J/cm²,
wherein the light is sequentially emitted and disrupted according to a frequency comprised between 0,001 and 10 Hz, and the ratio between the duration of light emission and the duration of light disruption being comprised between 45 and 80

A light source device which can be used in a method according to the invention comprises a light emitting element for emitting a light having a wavelength ranging from 435 to 520 nm, a power density greater than 20 mW/cm², the light source device may be configured to provide an effective fluence to any contaminating and/or pathogenic agent greater than 11 J/cm².

The dominant emission wavelength may range from 440 to 490 nm, more particularly from 450 to 460 nm.

The light source device may provide an effective fluence to any contaminating and/or pathogenic agent greater than 40 J/cm², preferably greater than 80 J/cm². The light emitting element may have a power density ranging from 20 to 400 mW/cm², more particularly a power density ranging from 21 to 150 mW/cm², and more particularly from 23 to 46 mW/cm², especially for use in any device able to contact the skin, wound or mucosa. The ratio between the effective fluence and the power density of the light emitting element may be greater than 1.7 preferably greater than 3.

The light emitting element may comprise at least one LED.

The light source device may comprise a power source providing electrical power to said light emitting element.

The power source may be a battery, a solar cell, or anything that can produce a power source.

The light source device may comprise at least one among a microchip processor, a control unit, a communication unit, an external port and a sensor.

It may be provided a light source assembly comprising a product adapted to be in contact with a support, in particular a surface, including the skin, mucosa or a wound and a light source device as described above connected to the product to provide light to at least one contaminating and/or pathogenic agent, preferably of the wound.

The product may be one among a dressing, a strip, a compression means, a band-aid, a patch, a gel, a film-forming composition and a rigid or flexible support (which could be inserted into body cavities like nose..), preferably a dressing.

The use of said light source device according or said light source assembly for reducing the contaminating and/or pathogenic agent's growth and number is described. The use of said light source device according or said light source assembly may be for the treatment of medium or surface decontamination, preferably through a photobiomodulation means. More specifically, the use of said light source device according or said light source assembly may be for the disinfection of wounds, mucosa, and skin, and also for the decontamination or disinfection of packing, wrapping, food products, and cleaning and/or domestic devices, preferably through a photobiomodulation means.

### BRIEF DESCRIPTION OF THE DRAWINGS

Figure 1 represents in a cross section view a photobiomodulation device used in the treatment of various supports or medium for promoting the growth and number reduction of contaminating and/or pathogenic agents in vitro or in vivo.
Figure 2 represents a histogram comparing the effect on colony count of E. *Coli* in blue light irradiation conditions (453 nm, defined as BLI) vs without irradiation (defined as control), and for irradicance values of 23 mW/cm².
Figure 3 represents a histogram comparing the effect on colony count of E. *Coli* in blue light irradiation conditions (453 nm, defined as BLI) Vs without irradiation (defined as control), and for irradicance values of 10 mW/cm².
Figure 4 represents two curves comparing the effect on colony count of K. *pneumoniae* in continuous blue light irradiation conditions (453 nm) vs discontinuous blue light irradiation conditions (453 nm, light exposure ratio of 50%, frequency of 0,02 Hertz) and for irradiance values of 23 mW/cm².
Figure 5 represents two curves comparing the effect on colony count of P. *aeruginosa* in continuous blue light irradiation conditions (453 nm) vs discontinuous blue light irradiation conditions (453 nm, light exposure ratio of 50%, frequency of 0,02 Hertz) and for irradiance values of 23 mW/cm².
Figure 6 represents two curves comparing the effect on colony count of E. *coli* in continuous blue light irradiation conditions (453 nm) vs discontinuous blue light irradiation conditions (453 nm, light exposure ratio of 50%, frequency of 0,02 Hertz) and for irradiance values of 23 mW/cm².

### DETAILED DESCRIPTION

The present invention will be described below relative to several specific embodiments. Those skilled in the art will appreciate that the present invention may be implemented in a number of different applications and embodiments and is not specifically limited in its application to the particular embodiment depicted herein.

For the purpose of the present invention, the following terms are defined.

The term "Wavelength" is the distance between two peaks of a wave. The symbol for wavelength is λ (lambda) and the unit of measurement is nanometers (nm).

The term "Dominant emission wavelength" is the wavelength or a narrow range of wavelengths the light source emits the majority of the time. The term "power" refers to the rate at which work is perform; the unit of power is Watt (W) and since the light output power is low it is expressed in milliwatts (mW).

The term "power density" or "light intensity", or "irradiance", or "exitance" is the power divided by the area of the target being illuminated by the light and is expressed in mW/cm².

The term "fluence" or "energy density" or "dose" expressed in Joules per cm² (J/cm²) is the product of power (mW) and time per spot size (cm²).

The term "transmitted fluence" is the fluence produced by the claimed light source, whereas the term "effective fluence" is the fluence actually received by the contaminating and/or pathogenic agent. Indeed, as will be further explained below, the effective fluence may be lower than the transmitted fluence depending, in particular, on the environment (medium or support) of the agent.

The term "photobiomodulation" is the ability of the light source device to have a biological effect on cells, or on contaminating and/or pathogenic agents, directly, which means without the need of any provisional product or composition to transpose or potentialize any biological effect engendered by the light source. This term can be distinguished from the term of "photodynamic therapy" which needs absolutely and every time the intervention of an intermediate product between the light source and the cells or contaminating and/or pathogenic agent to potentialize the biological effect of the light.

The term « contaminating and/or pathogenic agent » is intended to designate any microorganism such as bacteria, yeast or fungi, any virus or any organisms in contact with a skin, wound or mucosa such as parasite, louse, dust mite or worm.

By "contaminating agent", we intend to qualify any one of the « contaminating and/or pathogenic agent » listed above able to grow and proliferate on a specific support or in a specific medium (including an inert surface, a pharmaceutical composition, a biological surface, more particularly a skin, a mucosa or a wound, or any eventual food product, drink or beverage).

By "pathogenic agent", we intend to qualify any contaminating agent capable of inducing a disease or a biological trouble to an animal or a human being.

The term "support" is intended to designate any substrate or surface on which a contaminating and/or pathogenic agent can grow and proliferate, including an inert surface, a biological surface more particularly a skin, a mucosa, a wound, or any eventual food product or packing.

The term "medium" is intended to designate any environment in which bacteria can develop, grow and proliferate, including a pharmaceuticals composition, used waters, liquids, or the air.

The term "microorganism" is intended to designate bacteria, yeasts, and fungi.

The expression "growth and number reduction of contaminating and/or pathogenic agent" means that microorganisms, parasites and viruses, preferably bacteria, growth and number can be limited. This ability can be characterized by a bacterial reduction of at least 0.1 log or 20% measured in a suspension, in a wound dressing or on a support. In the case the method of measurement of the "growth and number reduction of contaminating and/or pathogenic agent" leads to a potential standard deviation in the obtained values, the results should be interpreted strictly. This means that every time a measured value could be lower than the defined threshold due to the variability of measurement; the expected antibacterial effect is not fulfilled.

A light source device comprising a light emitting element for emitting a light having the following characteristics:
a wavelength ranging from 435 to 520 nm, and
a power density greater than 20 mW/cm²,
the light source device (10) provides an effective fluence to any contaminating and/or pathogenic agent greater than 11 J/cm2 is described.

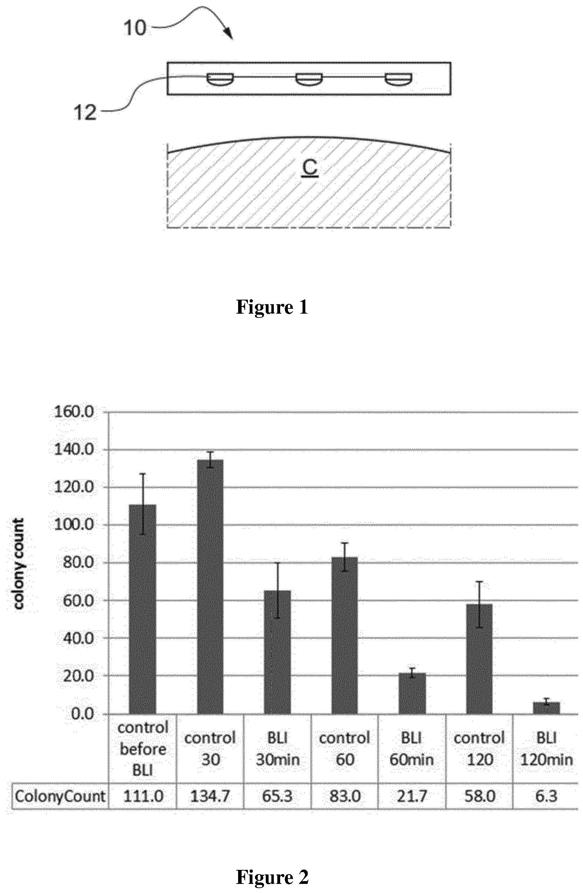

According to figure 1, a light source device 10 comprising a light emitting element 12 for emitting a light having a wavelength ranging from 435 to 520 nm is proposed. The light source device 10 is able to emit light at wavelengths within the range of 435 to 500 nm, preferably within a specific dominant emission wavelength of 440-490 nm and preferably within a specific dominant emission wavelength of 450-460 nm. More particularly, the chosen dominant emission wavelength may be 450 or 453 nm.

Furthermore, the light source device 10 may be configured to provide light to contaminating and/or pathogenic agent present on the support or medium C at irradiance and fluence (dose or energy density) able to at least inhibit this growth and number in said medium or support. The fluence at which light is provided to the contaminating and/or pathogenic agents present on the support or medium C corresponds to the specific conditions, particularly specific conditions of irradiance and exposure with a light source having a specific dominant emission wavelength ; allowing to obtain the unexpected technical effect with regard to the prior art. Indeed, it was observed that monitoring the irradiance of the provided light allows having a growth and number-reductive effect on irradiated contaminating and/or pathogenic agents.

The growth and number reduction of contaminating and/or pathogenic agents may be performed on any support or in any medium, *ex vivo* or *in vivo.* Indeed, contaminating and/or pathogenic agents may be present in liquid medium such as used waters or the air, on any inert surfaces or in human or animal tissues, in particular on wounds.

The light source device 10 may be configured to provide light at a specific fluence and power density to any support or medium, and for example to human or animal skin tissue or to *in vitro* contaminating and/or pathogenic agents such as bacteria to provide the growth and number-reductive effect. Thus, this light source device 10 is more particularly useful in wound treatment. Accordingly, the light source device would transmit the light onto the surface of a wound.

Depending on many interference means, as described above, disposed between the contaminating and/or pathogenic agent and the light source, the effective fluence of the light received by said agent may be lower than the fluence transmitted by the light emitting element. Indeed, it was also observed that a larger fluence has to be generally transmitted by the light emitting element 12 to provide a predetermined fluence of light to the contaminating and/or pathogenic agent on the support or medium C, i.e. an effective fluence of light adsorbed by the contaminating and/or pathogenic agent. Indeed, during the emission, a part of the light is adsorbed by other elements than the contaminating and/or pathogenic agent which induces a loss of light. Therefore, the light source device 10 may be configured to provide light at a transmitted fluence so that the contaminating and/or pathogenic agent receives a predetermined fluence (also called effective fluence). Depending on the elements that can be present between the light emitting element and the target contaminating and/or pathogenic agent, the attenuation or absorption effect of the light may lead to an attenuation ranging from 20% to 60% or from 30% to 50% of the energy density, preferably around 45%.

To obtain a growth and number-reductive effect of the contaminating and/or pathogenic agent, the irradiance or power density is of at least 20 mW/cm², particularly in the range from 20 to 400 mW/cm², more particularly a power density ranging from 21 to 150 mW/cm² and more particularly from 23 to 46 mW/cm².

The effective dose or fluence received by the contaminating and/or pathogenic agent, in particular a bacteria of a wound or a given surface of skin tissue, may be of at least 11 J/cm², and preferably from about 40 J/cm² to about 600 J/cm², or about 41 J/cm² to about 590 J/cm², or about 42 J/cm² to about 580 J/cm², or about 45 J/ cm² to about 570 J/ cm², about 50 J/ cm² to about 560 J/cm², or about 55 J/cm² to about 550 J/cm², or about 60 J/ cm² to about 540 J/cm², or about 65 J/cm² to about 530 J/cm², or about 70 J/ cm² to about 520 J/cm², or about 75 J/cm² to about 510 J/cm², or about 80 J/ cm² to about 500 J/cm², or any light dose in a range bounded by, or between, any of these values. Preferably, the effective fluence used to treat target contaminating and/or pathogenic agent, and preferably bacteria is greater than 40 J/cm², preferably greater than 80 J/cm².

As indicated above, the fluence (dose or energy density) notably depends on both irradiance (mW/cm²) and time. Therefore, obtaining the predetermined fluence may be accomplished by using a higher power light source, which may provide the needed energy in a shorter period of time, or a lower power light source may be used for a longer period of time. Thus, a longer exposure to the light may allow a lower power light source to be used, while a higher power light source may allow the treatment to be done in a shorter time.

The duration of radiation or light exposure administered to a medium or support containing the contaminating and/or pathogenic agent, may also vary. The exposure may range from at least 1 microsecond, 1 second, at least few seconds, or at least 30 minute, or at least 30, 31, 32, 33, 34, 35, 36, 37, 38, 39, 40, 41, 42, 43, 44, 45, 46, 47, 48 minutes; or up to about 5 hour, 4h, 3h, 2h, 1h or, for any amount of time in a range bounded by, or between, any of these values.

The light source device may be used in the growth and number reduction of contaminating and/or pathogenic agents under specific conditions. Particularly, it was observed that the growth and number-reductive effect occurs on contaminating and/or pathogenic agents when provided with an effective fluence greater than 11 J/cm² with a power density of about 20 mW/cm² during about 10 minutes to 2h. Preferably, it was observed that the growth and number-reductive effect occurs on contaminating and/or pathogenic agents when provided with an effective fluence greater than 40 J/cm² with a power density from about 23 mW/cm² to about 80 mW/cm² during about 30 minutes to 2h.

The light source device may be able to emit light continuously (for instance one time, providing specific fluence values), sequentially (for example many times, separated by defined latencies, providing specific fluence values) or by means of pulsations (for example one or many times, providing a specific fluence depending on opposite variations of irradiance and time of exposure values).

The ratio between the effective fluence and the power density of the light source device of the invention may begreater than 1.7 preferably greater than 3. The ratio between the effective fluence and the power density of the light source device characterizes the energy regarding the irradiance received by the contaminating and/or pathogenic agent, preferably the bacteria. It is an indicator qualifying the performance of the treatment.

For thermal issues, light source device may be configured to irradiate the contaminating and/or pathogenic agent either continuously or in pulses. Indeed, pulsed light irradiation will typically be preferred than continuous light if there are some thermal issues; indeed, light source provides heating. The decision whether to use constant irradiation of pulsed light irradiation depends on the exact application and on the total desired irradiation. When the light exposure depends on the duration of a cycled light, the net light time may be determined by the sum of the duration of each pulse.

The light emitting element 12 is a device able to perform photobiomodulation. An example of such a light emitting element 12 is a light-emitting diode (LED or OLED, preferably LED), a LASER, or a lamp (such as filament lamp, gaz lamp) which is able to emit light at wavelengths within the ranges of 435 to 520 nm and having preferably a dominant emission wavelength comprised between 450-460 nm, as well as at a dominant emission wavelength of 450 or 453 nm. In figure 1, the light emitting element 12 comprises three light-emitting diodes. Alternatively, the light emitting element 12 may comprise one or more light-emitting diode (or lamp) able to emit a blue light having a wavelength ranging from 435 to 520 nm, having preferably a dominant emission wavelength comprised between 450 to 460 nm or having a dominant emission wavelength of about 450 or 453 nm.

For supplying electricity to the light emitting element 12, the light source device 10 may comprise a power source connected to the light emitting element 12. The power source may comprise an electric cable to connect to a power grid or a battery scavenger. Alternatively, the power source may be a battery, or a solar cell, preferably a battery. The light source device 10 is compact and able to communicate with a smartphone or a tablet thanks to a wireless communication protocol (Bluetooth or Bluetooth smart or Bluetooth Low Energy, NFC, Wifi, Lifi, Lora, Zigbee, preferably Bluetooth Low Energy).

For controlling the light emitting element 12, the light source device 10 may comprise at least one among a LED Driver, a sensor, a microchip processor, a control unit, a communication unit and an external port, an antenna, a memory.

A sensor may allow the light source device 10 to measure parameters of the contaminating and/or pathogenic agent. These parameters may be for example the temperature and the oxygenation level of the treated surface.

The microchip processor or the control unit may allow the light source device 10 to monitor the supply of electricity to the light emitting element 12 to guarantee an optimum or desired light exposure. For example, the microchip processor or the control unit may control whether the light exposure is continuous, discontinuous or in cycles as well as the frequency and the duration of the pulses depending on predetermined parameters or live parameters such as values measured by a sensor of the light source device 10.

Furthermore, a communication unit may allow a user to recover data from or transmit data to the light source device 10. For example, data may be transmitted to a smartphone or any other external device, notably an external device comprising a screen to display information useful to the user. The communication unit may be configured for wireless transmission or wired communication. In the case of a wired communication, the light source device 10 may comprise an external port connected to the communication unit for data transmission. Alternatively, the communication unit may be configured for both wireless and wired communication.

Moreover, the light source device 10 may be included in a light source assembly (not shown) which comprises a product adapted to be in contact with a surface to be treated, for example the skin or a wound formed on the skin. In this case, the light source device 10 is connected to the product for providing light to at least one contaminating and/or pathogenic agent of the skin or the wound.

For improving light effect, the light source assembly may be adapted to dispose the light emitting element 12 in a position wherein the light emitting element 12 is facing the support. In other words, the light source assembly is also adapted to place the light emitting element on the facing page of the support.

Furthermore, the light source device 10 may be configured so that light is irradiated to the contaminating and/or pathogenic agent or to the support or medium through the product. In doing so, the light source device 10 can irradiate to the contaminating and/or pathogenic agent or to the support or medium, preferably the support without direct contact.

The light source assembly may be configured to allow setting or predetermining of the distance between the light emitting element 12 and the support. Indeed, light intensity decreases with the square of the distance from the source of the light. For example, light 1 meter away from a source is four times as intense as light 2 meters from the same source. Therefore, setting the distance between the light emitting element 12 and the support allows monitoring the irradiance and thus the fluence provided to the said surface. The distance between the light emitting element 12 and the support may be predetermined from 0 to 50 mm, and preferably 0 to 20 mm in the case of a wound dressing for example. This distance could be larger depending on the targeted use. For example in the food processing industry, the distance between the light emitting element 12 and the support may be of several centimeters in the case of a lamp used alone for example.

For setting or predetermining the distance between the light emitting element 12 and the support, the dimension of the product may be chosen to predetermine or set the distance between the light emitting element 12 and the support or medium. Alternatively or in combination, the light source assembly may further comprise an adjustable element for adjusting the distance between the light emitting element 12 and the support.

The light source device 10 may also be configured so that the light emitting element 12 may be selectively orientated to better target the contaminating and/or pathogenic agent to be irradiated. This orientation or homogenization of the light emitting element 12 allows the irradiation to be more adapted to the geometry and the characteristics of any contaminating and/or pathogenic agent or to the treated surface, support or medium. These advantages become even more significant when the light source device 10 comprises a plurality of light emitting elements 12. In this case, the light emitting elements 12 may be orientated independently from each other to widen the irradiated area.

Furthermore, the light source device 10 may comprise a lens for focusing the light onto the target the support or medium to make the irradiation more precise.

The product may be one among a dressing, a strip, a compression means, a Band-Aid, a patch, a gel and a rigid or flexible support, a film-forming composition or similar. Furthermore the product may be arranged so that the light emitting element 12 is disposed on the interior of the product or in its inferior or superior surface. The product adapted to contact the skin or a wound may be preferably a dressing. The dressing may comprise at least a hydrocolloid or an adhesive layer in contact with the skin or the wound.

The light source assembly may be of any size or shape. The assembly may be 8×8 cm (or more 20x20 cm for instance) in size. The assembly may be 4×4 cm in size. The product may comprise an interior layer comprising a mesh material and a tissue gel. The mesh material allows exudate from a wound to which the dressing is applied to be absorbed into the dressing whilst allowing the tissue gel to flow through it so that it can be absorbed by a wound being treated.

For allowing the light source assembly to be reusable while avoiding repetitive cleanup, the product may be disposable and interchangeable. In other words, the product may be configured to be separated from the light source device 10 so that a same light source device 10 can be used several times without the need of a cleanup. It also allows changing the electronic elements included in the light source device 10 for maintenance, for example for recharging the battery.

A method for inhibiting growth and reducing number of contaminating and/or pathogenic agent, preferably bacteria is also proposed. In this respect, the present invention is also directed to a method for inhibiting growth and reducing number of contaminating and/or pathogenic agent, said method comprising exposing said contaminating and/or pathogenic agent to the light source device 10 and the light source assembly described above.

Contaminating and/or pathogenic agent or the support or medium are irradiated with a light at wavelengths comprised between 435 to 520 nm, and preferably having a dominant emission wavelength comprised between 450 and 460 nm. More particularly, the chosen dominant emission wavelength may be of 450 or 453 nm. The method may be performed *in vivo* or *in vitro.* Bacteria, and more generally contaminating and/or pathogenic agent may be in culture or directly from a human or animal tissue.

To reduce their growth and number, contaminating and/or pathogenic agents may be irradiated to receive an effective fluence greater than 11 J/cm², preferably greater than 40 J/cm² and more preferably greater than 80 J/cm².

To reduce the growth and number of contaminating and/or pathogenic agents, light emitting source used in this method is greater than 20 mW/cm² and preferably comprised between 23 and 400mW/cm², more particularly a power density ranging from 21 to 150 mW/cm² and more particularly from 23 to 46 mW/cm².

More generally, the irradiation of light performed in this method may be set using all the different values of fluence, power intensity and time described above for the light source device 10 and the light source assembly.

This method allows to benefit from the same effects as described above for the light source device 10 and the light source assembly. Particularly, the present method allows to obtain the unexpected technical effect of light consisting in at least inhibiting growth and reducing number of contaminating and/or pathogenic agent.

In particular, the method according to the invention is very useful for reducing growth and number of contaminating and/or pathogenic agents, for the treatment of fluid or liquid medium such as respectively the air or used waters, for surface decontamination or disinfection of wounds, mucosa and skin, or such as packing, wrapping, food products, and cleaning and/or domestic devices preferably through a photobiomodulation means. More specifically, the method according to the invention is very useful for reducing growth and number of contaminating and/or pathogenic agents of wound, skin or mucosa.

In a specific embodiment the present invention also discloses a method for inhibiting growth and reducing number of contaminating and/or pathogenic agent comprising exposing said contaminating and/or pathogenic agent to a light source device (10) comprising a light emitting element (12) for emitting a light having a wavelength ranging from 435 to 520 nm, , having a power density greater than 20 mW/cm² and the light source device (10) providing an effective fluence to any contaminating and/or pathogenic agent greater than 11 J/cm², wherein the light is sequentially emitted and disrupted according to a frequency comprised between 0,001 and 10 Hz, and the ratio between the duration of light emission and the duration of light disruption being comprised between 45 and 80.

It is indeed of the merit of the inventors to have discovered that the inhibition of growth and/or the reduction of the number of contaminating and/or pathogenic agent is unexpectedly enhanced when the contaminating and/or pathogenic agent is exposed discontinuously to a light having a wavelength ranging from 435 to 520 nm, compared to a continuous exposure. In particular, the enhanced inhibition of growth and/or reduction of the number of contaminating and/or pathogenic agent can be obtained by subjecting the agent to the same light having a wavelength ranging from 435 to 520 nm, and adjusting either the power density of each sequence of irradiation or the total duration of exposure to the light so that the total effective fluence received by the agent during the whole treatment remains the same.

By "discontinuous exposure", it should be understood that the light is sequentially emitted and disrupted at least twice, preferably at least 10 times, more preferably at least 15 times. "Discontinuous exposure" also means any cycled or pulsed exposure, that should be understood as the sequential emission and disruption of light defined by a specific frequency and a specific period of time.

In a particular embodiment, each sequence of emission/disruption of light lasts for a period of time which can be an attosecond, a femtosecond, a picosecond, a nanosecond, a microsecond, a millisecond, a second, a minute, one hour, one day. Each sequence can also be defined by its frequency in Hertz, milli-Hertz, micro-Hertz, kilo-Hertz, mega-Hertz, giga-Hertz or tera-Hertz, the frequency being the inverse of the period. According to a specific embodiment, each sequence is characterized by a frequency comprised between 0.001 and 10 Hz.

The sequences of emission/disruption may be symmetrical in the sense that the duration of light emission and the duration of light disruption is the same. Alternatively, the sequences of emission/disruption may be asymmetrical in the sense that the duration of light emission and the duration of light disruption is different.

Each sequence of emission/disruption can be defined by a "light exposure ratio" (or as "duty cycle"), corresponding to the ratio between the duration of light emission and the duration of light disruption. The light exposure ratio is expressed as a percentage and is comprised between 0 and 100%, the ends of the range (0% and 100%) being excluded. A light exposure ratio close to 0% means that the light is disrupted during almost the entire sequence. A light exposure ratio close to 100% means that the light emits during almost the entire sequence. According to a specific embodiment, the light exposure ratio could be comprised between 45 and 80.

A light source device is described for use for the *in vivo* growth and number reduction of microorganism and/or virus on a support or in a medium.

Alight source assembly containing a light source device is described for use for the *in vivo* growth and number reduction of contaminating and/or pathogenic agent on a support or in a medium

Alight source device or to the light source assembly described above is described for use in reducing contaminating and/or pathogenic agent growth and number, and in particular for a use as a bactericide.

The invention will be illustrated further by the following examples:

### Example 1: Effect of the blue light on the growth and number reduction of S. aureus (comparative example)

### Bacteria cells in suspension

1 mL of a *S*. *aureus* (ATCC 6538) solution at a concentration of 1.5 to 5×10⁷ CFU/ml were inoculated in Petri dishes comprising 9 mL of a mixture of 50% of buffered peptone water (0.1%) and 50% foetal veal serum (Simulated Wound Fluid or SWF), bacteria concentration was 1.5×10⁶ CFU/mL in the Petri dish.

Then, the Petri dishes inoculated with the bacteria are treated by a blue light.

### Light treatment

For the light treatment, OSRAM GD PSLR31.13 is used, with dominant emission wavelength of 450 nm (blue light). Dressings were directly irradiated with a power density of 23 or 46mW/cm².

### Bacterial enumeration

Bacterial enumeration was conducted before light treatment, and after exposure to light to observe the reductive effect of light treatment on bacterial growth and number.

### Results

**Table 1: bacterial growth and number reduction observed after irradiation of S.aureus cells in suspension in SWF**

| Power density (mW/cm²) | Effective Fluence (J/cm²) | Time of exposure (min) | Ratio⁽¹⁾ | Bacterial reduction (Log) | Bacterial reduction (%) |
|---|---|---|---|---|---|
| 23 | 166 | 120 | 7.21 | 0,26 | 45 |
| 23 | 248 | 180 | 10.78 | 0,74 | 81 |
| 23 | 331 | 240 | 14.390 | 1,15 | Superior to 90 |
| 23 | 414 | 300 | 18 | 1,5 | Superior to 90 |
| 46 | 331 | 120 | 7.21 | 5,58 | Superior to 99 |

| | | | | | |
|---|---|---|---|---|---|
| ⁽¹⁾ Ratio between the effective fluence and the power density | | | | | |

The results show that the exposure of *S. aureus* to blue light (450 nm) with energy densities of greater than 41 J/cm², significantly inhibits bacterial growth and number and proliferation which well shows that such irradiation with blue light can be used to inhibit bacterial development on solid supports, and in particular on wounds and injuries.

### Example 2: Effect of the blue light on the growth and number reduction of P. aeruginosa (comparative example)

### Bacteria cells immobilized in wound dressings

*P. aeruginosa* (ATCC 15442) at a concentration of 1.5 to 5×10⁷ CFU/mL were inoculated on the surface of pre-wetted dressings. The concentration of bacteria was about 5×10⁶ CFU/dressing.

Then, the dressings inoculated with the bacteria are treated by a blue light.

### Light treatment

For the light treatment, OSRAM GD PSLR31.13 is used, with dominant emission wavelength of 450 nm (blue light). Dressings were directly irradiated with a power density of 23 or 46 mW/cm².

### Bacterial enumeration

Bacterial enumeration was conducted before light treatment, and after exposure to light to observe the reductive effect of light treatment on bacterial growth and number.

### Results

**Table 2: bacterial number and growth reduction observed after irradiation of inoculated dressings with P. aeruginosa**

| power density (mW/cm ²) | Effectiv e Fluence (J/cm²) | Time of exposure (mins) | Ratio⁽¹⁾ | Bacterial reduction (Log) | Bacterial reduction (%) |
|---|---|---|---|---|---|
| 23 | 10 | 7,5 | 0.43 | 0.02 | 4 |
| 23 | 41 | 30 | 1.78 | 0,14 | 27 |
| 23 | 83 | 60 | 3.60 | 0,39 | 59 |
| 23 | 166 | 120 | 7.21 | 1,04 | Superior to 90 |
| 23 | 414 | 300 | 18 | 4,56 | Superior to 99 |
| 46 | 166 | 60 | 3.60 | 3,53 | Superior to 99 |
| 46 | 414 | 150 | 9 | 4,74 | Superior to 99 |

| | | | | | |
|---|---|---|---|---|---|
| (1) Ratio between the effective fluence and the power density | | | | | |

The results show that the exposure of *P. aeruginosa* to blue light (450 nm) with energy densities greater than 41 J/cm², significantly reduces bacterial growth and number which well shows that such irradiation with blue light can be used to inhibit bacterial development on solid supports, and in particular on wounds and injuries.

In conclusion, the results exhibit that the exposure of bacteria (whatever the considered species, Gram-positive or Gram-negative) to blue light (specifically at 450 nm or 453 nm) with energy densities greater than 11 J/cm², preferably greater than 40 J/cm² (more precisely, 41 J/cm²), and irradiance values greater than 20 mW/cm², significantly reduces the bacterial growth and number.

### Example 3: Effect of the blue light on the growth and number of E. coli (comparative example)

### Bacterial culture

0.5 mL of an *Escherichia coli* (strain K12) (Taxon identifier: 83333) solution at a concentration of 1×10⁶ CFU/mL in NaCl 0.9% were inoculated in 4.5 mL nutrient broth (8 g/L (Merck)). Afterwards, a dilution series is prepared and then after irradiation by a blue light, bacteria were seeded on plates and number of colonies was counted 24 hours later.

### Light treatment

For the light treatment, Lumileds Luxeon Rebel LXML-PR01-0275 from Koninklijke Philips N.V. (Eindhoven/Netherlands) was used, with a dominant emission wavelength of 453 nm (blue light).

Suspensions were directly irradiated with a power density of 10 or 23 mW/cm².

### Bacterial enumeration

Bacterial enumeration was conducted before light treatment, and after incubation to observe the inhibitory effect of light treatment on bacterial growth and number.

### Results

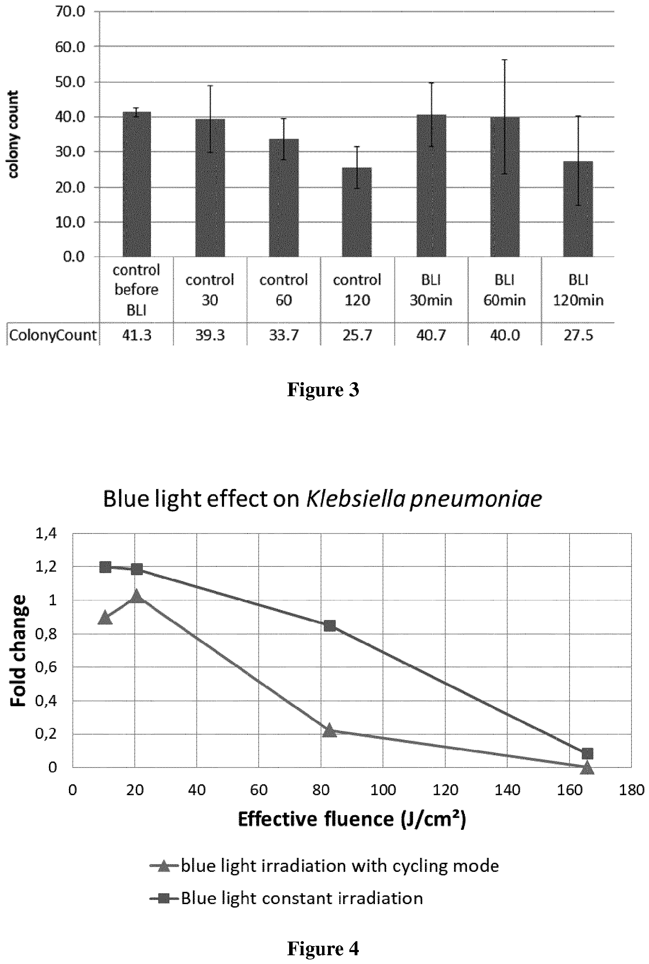

Results are shown in figure 2 and 3.

### Nb:

- BLI means Blue light irradiation
- Control does not receive any light irradiation whatever the exposure time considered
- Numbers following BLI or control mention, express time (with exposure to blue light (BLI) or without exposure (control)) after which colony count has been made.

**Table 3: Correspondence between time of exposure, power density used and fluence definition for each condition**

| power density (mW/cm²) | Time of exposure | Effective Fluence (J/cm²) |
|---|---|---|
| 23 | 30 min | 41 |
| 23 | 60 min | 83 |
| 23 | 120 min | 166 |
| 10 | 30 min | 18 |
| 10 | 60 min | 36 |
| 10 | 120 min | 72 |

Figure 2 represents a histogram comparing the effect on colony count of E. *Coli* in blue light irradiation conditions (453 nm, defined as BLI) Vs without irradiation (defined as control), and for irradicance values of 23 mW/cm².

Figure 3 represents a histogram comparing the effect on colony count of E. *Coli* in blue light irradiation conditions (453 nm, defined as BLI) Vs without irradiation (defined as control), and for irradicance values of 10 mW/cm².

The results show that the exposure of *E. coli* to blue light (453 nm) with a device having the irradiance feature of 10 and 23 mW/cm² induce respective different issues too. More precisely, blue light irradiation of *E. coli* at 23 mW/cm² (fig. 2) induces a drastic reduction of the number of colonies counted whatever the time of exposure. On the contrary, there is no significant effect on the colony number measured between *E. coli* treated with blue light (10 mW/cm²) and control group for the same time of exposure (fig. 3).

Indeed, examples 1 to 3 show that a device comprising a light emitting element for emitting a light having the following characteristics:
a wavelength ranging from 435 to 520 nm, and
a power density greater than 20 mW/cm²,
the light source device provides an effective fluence to any contaminating and/or pathogenic agent greater than 11 J/cm² exhibit a specific and surprising effect on the growth and number reduction of contaminating and/or pathogenic agent, preferably bacteria, whatever the specie (Gram-positive or Gram-negative) of bacteria.

### Example 4: Effect of the cycled blue light on the growth and number of K. Pneumoniae, P. Aeruginosa and E. Coli. (comparative example)

### Bacterial culture

0.5 mL of an *Escherichia coli* (strain K12) (Taxon identifier: 83333) solution at a concentration of 1×10⁶ CFU/mL in NaCl 0.9% were inoculated in 4.5 mL nutrient broth (8 g/L (Merck)). Afterwards, a dilution series is prepared and then after irradiation by a blue light, bacteria were seeded on plates and number of colonies was counted 24 hours later.

### Light treatment

For the light treatment, Lumileds Luxeon Rebel LXML-PR01-0275 from Koninklijke Philips N.V. (Eindhoven/Netherlands) were used, with a dominant emission wavelength of 453 nm (blue light).

Suspensions were directly irradiated:
- with a continuous exposure to light having a power density of 23 mW/cm² and
- with a discontinuous exposure to light with a power density of 23 mW/cm² characterized by a light exposure ratio of 50% and a frequency of 0,02 Hertz, and a total duration of exposure doubled compared to the continuous exposure.

The light source device provides an effective fluence to any contaminating and/or pathogenic agent greater than 11 J/cm².

### Bacterial enumeration

Bacterial enumeration was conducted before light treatment, and after incubation to observe the inhibitory effect of light treatment on bacterial growth and number.

### Results

Results are presented in figure 4, 5 and 6.

Figure 4 represents two curves comparing the effect on colony count of K. *pneumoniae* in continuous blue light irradiation conditions (453 nm) Vs discontinuous blue light irradiation conditions (453 nm, light exposure ratio of 50%, frequency of 0,02 Hertz) and for irradiance values of 23 mW/cm².

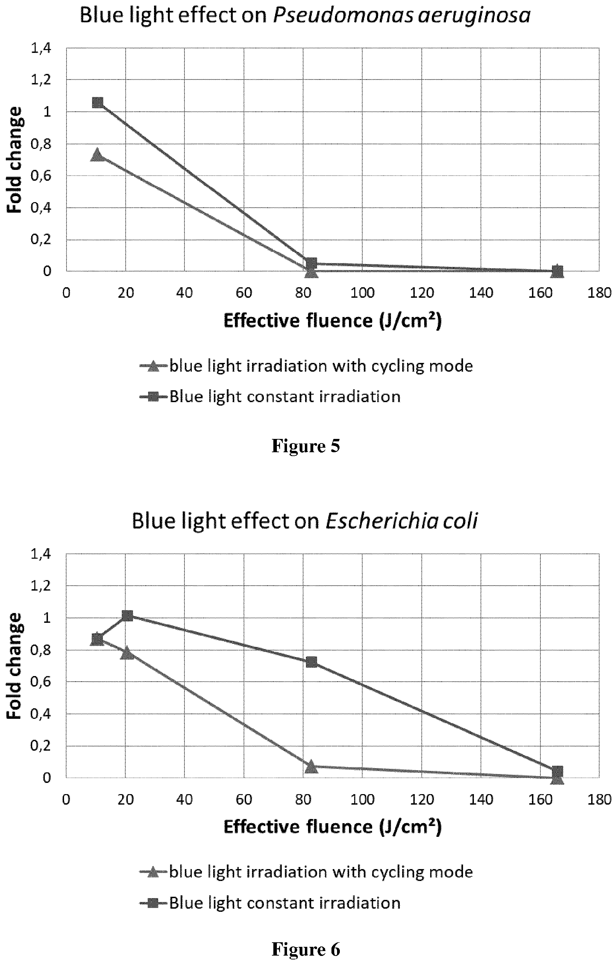

Figure 5 represents two curves comparing the effect on colony count of P. *aeruginosa* in continuous blue light irradiation conditions (453 nm) Vs discontinuous blue light irradiation conditions (453 nm, light exposure ratio of 50%, frequency of 0,02 Hertz) and for irradiance values of 23 mW/cm².

Figure 6 represents two curves comparing the effect on colony count of E. *coli* in continuous blue light irradiation conditions (453 nm) Vs discontinuous blue light irradiation conditions (453 nm, light exposure ratio of 50%, frequency of 0,02 Hertz) and for irradiance values of 23 mW/cm².

The results show that the exposure of K. *pneumoniae,* P. *aeruginosa* or E. *coli* to discontinuous blue light with a device having a power density of 23 mW/cm² and a duration of treatment doubled, induces an enhanced reduction of the number of colony counted compared to the same bacteria treated continuously with the same blue light.

### Example 5: Effect of the blue light on the growth and number reduction of S. aureus (comparative example)

### Bacteria cells immobilized on rigid and inert metal plate (support) [adaptation of the French standard NF EN 13697 : 2015]

*S. aureus* (ATCC 6538) at a concentration of 1.5 to 5×10⁸ CFU/mL were inoculated on the surface of a rigid and inert metal plate.

Then, the support inoculated with the bacteria is treated by a blue light.
Samples: n = 3

### Light treatment

For the light treatment, OSRAM GD PSLR31.13 is used, with dominant emission wavelength of 450 nm (blue light). Support is directly irradiated with a power density of 23 or 80 mW/cm².

### Bacterial enumeration

Bacterial enumeration was conducted before light treatment, and after exposure to light to observe the reductive effect of light treatment on bacterial growth and number.

**Table 4: bacterial growth and number reduction observed after irradiation of S. aureus cells on a support**

| Power density (mW/cm²) | Effective fluence (J/cm²) | Time of exposure (mins) | Ratio⁽¹⁾ | Bacterial reduction (Log) | Bacterial reduction (%) |
|---|---|---|---|---|---|
| 80 | 24 | 5 | 0.3 | 0.16 +/-0.11 | 27 +/- 20 |
| 23 | 41 | 30 | 1.78 | 0.37 +/-0.11 | 58 +/- 20 |
| 23 | 166 | 166 | 7.2 | 1.01 +/-0.26 | Superior to 99 |
| 80 | 240 | 240 | 3 | 2.43 +/-0.39 | Superior to 99 |

| | | | | | |
|---|---|---|---|---|---|
| ⁽¹⁾ Ratio between the effective fluence and the power density | | | | | |

Contrary to the tests conducted in example 1 and/or 2, the results obtained with the present method are expressed with a standard deviation in the logarithmic scale reduction.

This however does not change the interpretation of the results:
The reduction of bacterial growth and number observed for an effective fluence of 24 J/cm² and a power density of 80 mW/cm², and so, for a measured ratio of 0.3 are clearly non-significant as stated by the definition given of "growth and number reduction of contaminating and/or pathogenic agent", whatever the method used.

The results show that the expected technical effect for growth and number reduction of bacteria is obtained for ratios of at least of 1.7 or greater and preferably at least of 3.

### Example 6: Effect of the pulsed blue light on the growth and number reduction of S. aureus and P. aeruginosa

### Bacteria cells immobilized on rigid and inert metal plate (Support) [adaptation of the French standard NF EN 13697 : 20151

*S. aureus* (ATCC 6538) or *P. aeruginosa* (ATCC 15442) at a concentration of 1.5 to 5×10⁸ CFU/mL were inoculated respectively on the surface of a rigid and inert metal plate.

Then, the inoculated support with the bacteria is treated by a pulsed blue light. Conditions of this used pulsed blue light are: frequency of 3 Hz, duty cycle of 80%.
Samples: n = 1

### Light treatment

For the light treatment, OSRAM GD PSLR31.13 is used, with a dominant emission wavelength of 450 nm (blue light). Support is directly irradiated with a power density of 23, 198, 300 or 400 mW/cm².

### Bacterial enumeration

Bacterial enumeration was conducted before light treatment, and after exposure to light to observe the reductive effect of light treatment on bacterial growth and number.

**Table 5: Bacterial growth and number reduction observed after irradiation of S. aureus cells on a support**

| Power density (mW/cm²) | Effective fluence (J/cm²) | Time of exposure (mins) | Ratio⁽¹⁾ | Bacterial reduction (Log) | Bacterial reduction (%) |
|---|---|---|---|---|---|
| 23 | 41 | 30 | 1.78 | 0.67 | Superior to 90 |
| 198 | 166 | 14 | 0.84 | 2.22 | Superior to 99 |
| 133 | 240 | 30 | 1.8 | 1.65 | Superior to 99 |
| 300 | 240 | 13.5 | 0.8 | 4.11 | Superior to 99 |

| | | | | | |
|---|---|---|---|---|---|
| ⁽¹⁾ Ratio between the effective fluence and the power density | | | | | |

**Table 6: Bacterial growth and number reduction observed after irradiation of P.aeruginosa cells on a support**

| Power density (mW/cm²) | Effective fluence (J/cm²) | Time of exposure (mins) | Ratio⁽¹⁾ | Bacterial reduction (Log) | Bacterial reduction (%) |
|---|---|---|---|---|---|
| 23 | 41 | 30 | 1.78 | 1.12 | Superior to 99 |
| 198 | 166 | 14 | 0.84 | 2.27 | Superior to 99 |
| 133 | 240 | 30 | 1.8 | 2.82 | Superior to 99 |
| 400 | 240 | 10 | 0.6 | 4.94 | Superior to 99 |

| | | | | | |
|---|---|---|---|---|---|
| ⁽¹⁾ Ratio between the effective fluence and the power density | | | | | |

The results exhibit a significant and drastic effect over the reduction of bacteria growth and number when submitted to an exposure of a pulsed blue light.

The results seem equivalent between each kind of tested bacteria strain. Compared to the results obtained in example 5 (continuous light), the pulsed blue light shows an enhanced effect in the reduction of bacteria growth and number whatever the ratio for a specific and defined effective fluence used.

## Claims

1. An *in-vitro* method for inhibiting growth and reducing number of contaminating and/or pathogenic agents, the method comprising the step of:
exposing the contaminating and/or pathogenic agents with a light source device (10) comprising a light emitting element (12) for emitting a blue light having a wavelength ranging from 435 to 520 nm, having a power density greater than 20 mW/cm² and
the light source device (10) being configured to provide an effective fluence to the contaminating agent and/or pathogenic agents greater than 11 J/cm²,
wherein the light is sequentially emitted and disrupted according to a frequency comprised between 0,001 and 10 Hz, and the ratio between the duration of light emission and the duration of light disruption being comprised between 45 and 80.

2. The method of claim 1, wherein the dominant emission wavelength ranges from 440 to 490 nm, more particularly from 450 to 460 nm,

3. The method of claim 1 or 2 , wherein the light source device (10) is configured to provide an effective fluence to the contaminating and/or pathogenic agent greater than 40 J/cm², preferably greater than 80 J/cm².

4. The method according to any one of claims 1 to 3, wherein said light emitting element (12) comprises at least one LED.

5. The method according to any one of claims 1 to 4, further comprising a power source providing electrical power to said light emitting element (12).

6. The method according to any one of claims 1 to 5, wherein the contaminating and/or pathogenic agent is a microorganism such as bacteria, yeasts or fungi, preferably bacteria,

7. The method according to any one of claims 1 to 5, wherein the contaminating and/or pathogenic agent is a bacteria, in particular a Gram-positive or Gram-negative bacteria, preferably chosen from *S*. *aureus* and *P. aeruginosa.*

8. The method according to any one of claims 1 to 7 for the treatment of medium, or surface decontamination, preferably through a photobiomodulation means.

9. The method according to any one of claims 1 to 7 for the decontamination or disinfection of packing, wrapping, food products, and cleaning and/or domestic devices, preferably through a photobiomodulation means.

## Patentansprüche

1. *In-vitro*-Verfahren zur Hemmung des Wachstums und zur Verringerung der Anzahl von kontaminierenden und/oder pathogenen Agenzien, wobei das Verfahren die folgenden Schritte umfasst:
Exponieren der kontaminierenden und/oder pathogenen Agenzien mit einer Lichtquellenvorrichtung (10), die ein lichtemittierendes Element (12) zum Emittieren eines blauen Lichts mit einer Wellenlänge im Bereich von 435 bis 520 nm umfasst, mit einer Leistungsdichte von mehr als 20 mW/cm² und
wobei die Lichtquellenvorrichtung (10) so konfiguriert ist, dass sie eine effektive Fluenz für das kontaminierende Agens und/oder die pathogenen Agenzien von mehr als 11,1 J/cm² bereitstellt,
wobei das Licht nacheinander mit einer Frequenz zwischen 0,001 und 10 Hz emittiert und unterbrochen wird, und das Verhältnis zwischen der Dauer der Lichtemission und der Dauer der Lichtunterbrechung zwischen 45 und 80 liegt.

2. Verfahren nach Anspruch 1, wobei die dominante Emissionswellenlänge im Bereich von 440 bis 490 nm, insbesondere von 450 bis 460 nm, liegt.

3. Verfahren nach Anspruch 1 oder 2, wobei die Lichtquellenvorrichtung (10) so konfiguriert ist, dass sie dem kontaminierenden und/oder pathogenen Agens eine effektive Fluenz von mehr als 40 J/cm², vorzugsweise mehr als 80 J/cm², zuführt.

4. Verfahren nach einem der Ansprüche 1 bis 3, wobei das lichtemittierende Element (12) mindestens eine LED umfasst.

5. Verfahren nach einem der Ansprüche 1 bis 4, das ferner eine Stromquelle umfasst, die das lichtemittierende Element (12) mit elektrischer Energie versorgt (12).

6. Verfahren nach einem der Ansprüche 1 bis 5, wobei das kontaminierende und/oder pathogene Agens ein Mikroorganismus wie Bakterien, Hefen oder Pilze ist, vorzugsweise ein Bakterium.

7. Verfahren nach einem der Ansprüche 1 bis 5, wobei das kontaminierende und/oder pathogene Agens ein Bakterium ist, insbesondere ein Gram-positives oder Gram-negatives Bakterium, vorzugsweise ausgewählt aus *S. aureus* und *P. aeruginosa.*

8. Verfahren nach einem der Ansprüche 1 bis 7 zur Behandlung eines Mediums oder zur Oberflächendekontamination, vorzugsweise durch ein Photobiomodulationsmittel.

9. Verfahren nach einem der Ansprüche 1 bis 7 zur Dekontamination oder Desinfektion von Verpackungen, Umhüllungen, Lebensmitteln und Reinigungs- und/oder Haushaltsgeräten, vorzugsweise durch ein Photobiomodulationsmittel.

## Revendications

1. Procédé *in vitro* pour inhiber la croissance et réduire le nombre d'agents contaminants et/ou pathogènes, le procédé comprenant l'étape de :
exposer les agents contaminants et/ou pathogènes à un dispositif (10) de source de lumière comprenant un élément électroluminescent (12) pour émettre une lumière bleue présentant une longueur d'onde dans la plage de 435 à 520 nm, présentant une densité de puissance de plus de 20 mW/cm² et
le dispositif (10) de source de lumière étant configuré pour fournir une fluence effective à l'agent contaminant et/ou aux agents pathogènes de plus 11 J/cm²,
dans lequel la lumière est émise et interrompue de manière séquentielle selon une fréquence comprise entre 0,001 et 10 Hz, et le rapport entre la durée d'émission de lumière et la durée d'interruption de lumière étant compris entre 45 et 80.

2. Procédé selon la revendication 1, dans lequel la longueur d'onde d'émission dominante est comprise dans la plage de 440 à 490 nm, plus particulièrement de 450 à 460 nm.

3. Procédé selon la revendication 1 ou 2, dans lequel le dispositif (10) de source de lumière est configuré pour fournir une fluence efficace à l'agent contaminant et/ou pathogène de plus 40 J/cm², de préférence de plus 80 J/cm².

4. Procédé selon l'une quelconque des revendications 1 à 3, dans lequel ledit élément électroluminescent (12) comprend au moins une LED.

5. Procédé selon l'une quelconque des revendications 1 à 4, comprenant en outre une source d'alimentation fournissant de l'énergie électrique audit élément électroluminescent (12).

6. Procédé selon l'une quelconque des revendications 1 à 5, dans lequel l'agent contaminant et/ou pathogène est un microorganisme tel qu'une bactérie, des levures ou des champignons, de préférence une bactérie.

7. Procédé selon l'une quelconque des revendications 1 à 5, dans lequel l'agent contaminant et/ou pathogène est une bactérie, en particulier une bactérie Gram-positif ou Gram-négatif, de préférence choisie parmi *S*. *aureus* et *P. aeruginosa.*

8. Procédé selon l'une quelconque des revendications 1 à 7 pour le traitement de milieu, ou la décontamination de surface, de préférence par le biais d'un moyen de photobiomodulation.

9. Procédé selon l'une quelconque des revendications 1 à 7 pour la décontamination ou la désinfection de paquets, d'emballages, de produits alimentaires et de dispositifs de nettoyage et/ou domestiques, de préférence par le biais d'un moyen de photobiomodulation.
